(19) Europäisches Patentamt
European Patent Office
Office européen des brevets

(11) EP 1 707 186 A1

(12) # DEMANDE DE BREVET EUROPEEN

(43) Date de publication:
**04.10.2006 Bulletin 2006/40**

(51) Int Cl.:
***A61K 8/44*** (2006.01) ***A61K 8/84*** (2006.01)
***A61Q 5/06*** (2006.01)

(21) Numéro de dépôt: **06290508.8**

(22) Date de dépôt: **31.03.2006**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **31.03.2005 FR 0503151**

(71) Demandeur: **L'ORÉAL**
**75008 Paris (FR)**

(72) Inventeurs:
- **Gourlaouen, Luc**
  **92600 Asnières (FR)**
- **Plos, Grégory**
  **158-0097 Tokyo (JP)**

(74) Mandataire: **Dossmann, Gérard**
**Bureau Casalonga & Josse**
**Bayerstrasse 71/73**
**80335 München (DE)**

(54) **Composition comprenant un monomère électrophile et un polymère conducteur, et procédés de traitement cosmétique des fibres kératiniques**

(57) La présente invention concerne une composition comprenant dans un milieu cosmétiquement acceptable, au moins un monomère électrophile et au moins un polymère conducteur, et son utilisation pour le traitement cosmétique des fibres kératiniques.

Elle concerne aussi un procédé de traitement cosmétique des fibres kératiniques mettant en oeuvre ladite composition.

EP 1 707 186 A1

Printed by Jouve, 75001 PARIS (FR)

## Description

La présente invention est relative à une composition comprenant au moins un monomère électrophile et au moins un polymère conducteur, à son utilisation pour le traitement cosmétique des fibres kératiniques et à un procédé de traitement cosmétique mettant en oeuvre une telle composition.

Dans le domaine de la cosmétique, on cherche à modifier les propriétés superficielles des fibres kératiniques telles que les cheveux, par exemple pour apporter aux cheveux un effet conditionnant comme la douceur, ou de la brillance. Pour ce faire, on utilise généralement des compositions cosmétiques à base d'agents de conditionnement tels que des silicones ou des polymères ayant une forte affinité pour les fibres kératiniques, en particulier pour les cheveux.

Cependant, ces agents de conditionnement ont tendance à s'éliminer au cours de lavage avec des shampoings, rendant nécessaire le renouvellement des applications des compositions sur les cheveux.

Pour accroître la rémanence de dépôt de polymères, il peut être envisagé d'effectuer une polymérisation radicalaire de certains monomères directement sur les cheveux. Toutefois, on constate une forte dégradation des fibres capillaires liées probablement aux amorceurs de polymérisation et les cheveux ainsi traités sont difficilement démêlables.

La Demanderesse a trouvé de manière surprenante qu'en utilisant l'association d'au moins un polymère conducteur à au moins un monomère électrophile tel que décrit ci-dessous, il était possible d'obtenir un conditionnement et une brillance améliorés des cheveux, et ce de manière durable.

En effet, une composition comprenant une telle association permet de maintenir la douceur et la brillance apportées à la chevelure par ladite composition, et ceci sans ré-application, même après plusieurs lavages de la chevelure.

L'application d'une composition comprenant une telle association conduit à la formation in situ d'un revêtement lubrifiant et brillant rémanent, notamment aux shampoings.

En outre, ce revêtement est homogène et lisse et présente une excellente adhésion sur les fibres kératiniques.

Ladite association apporte en particulier à la chevelure une brillance plus intense. Utilisée en présence de colorants ou de pigments, elle permet d'obtenir une brillance supérieure et durable des cheveux colorés qui n'ont généralement plus aucun brillant après le premier shampoing.

L'invention a donc pour objet une composition comprenant dans un milieu cosmétiquement acceptable, au moins un monomère électrophile et au moins un polymère conducteur.

Un autre objet de la présente invention consiste en l'utilisation de ladite composition pour le traitement cosmétique des fibres kératiniques, et plus particulièrement des cheveux.

L'invention a encore pour objet un procédé de traitement cosmétique des fibres kératiniques, et plus particulièrement des cheveux, mettant en oeuvre ladite composition.

D'autres objets, caractéristiques, aspects et avantages de l'invention apparaîtront encore plus clairement à la lecture de la description et des exemples qui suivent.

Selon l'invention, la composition comprend dans un milieu cosmétiquement acceptable, au moins un monomère électrophile et au moins un polymère conducteur.

Par "polymère conducteur", on entend au sens de la présente invention, une structure moléculaire dans laquelle le ou les monomères présentent une forte délocalisation électronique et dont la disposition dans le squelette du polymère permet aux orbitales $\pi$ de se recouvrir. Cette caractéristique chimique se traduit par un phénomène de conduction électrique qui s'accompagne ou non d'un phénomène d'absorption dans le spectre UV-visible, voire dans l'infrarouge.

Par polymère conducteur absorbant dans le visible, on entend au sens de la présente invention, tout polymère conducteur présentant une absorbance non nulle dans le domaine de longueur d'ondes allant de 400 à 800 nm, même si les maxima d'absorption du polymère se situent en dehors de cette gamme.

Les polymères conducteurs mis en oeuvre dans le cadre de la présente invention sont de préférence des polymères conducteurs solubles dans le milieu cosmétique approprié à l'application. On dit que le polymère est soluble dans le milieu lorsqu'il forme un liquide limpide isotrope à 25°C dans le milieu comprenant de l'eau ou un mélange eau/solvant ; ceci étant obtenu dans tout ou partie d'une gamme de concentration comprise entre 0,001 et 50 % en poids de polymère conducteur.

De plus, les polymères présentent de manière avantageuse, une conductivité comprise entre $10^{-5}$ et $5.10^{5}$ siemens/cm, plus particulièrement comprise entre $10^{-3}$ et $10^{5}$ siemens/cm, et de préférence comprise entre $10^{-1}$ et $10^{4}$ siemens/cm.

La conductivité est mesurée à l'aide d'un générateur de courant (RM2 Test Unit commercialisé par la société Jandel) muni d'une tête de mesure dite quatre pointes (Universal four-point probes commercialisé par la société Jandel). Les quatre pointes alignées et distantes du même espacement d sont appliquées par simple pression sur l'échantillon à analyser. Un courant I est injecté par les pointes externes à l'aide de la source de courant créant ainsi une variation de potentiel. La tension U est mesurée entre les deux pointes internes reliées au voltmètre du générateur de courant.

Dans cette configuration la conductivité $\sigma$ de l'échantillon exprimée en S/cm est donnée par l'expression suivante :

$$\sigma = (K \times I) / (U \times e)$$

avec :

K : coefficient dépendant de la position des contacts sur la surface de l'échantillon. Lorsque les pointes sont alignées et équidistantes, K est égal à : II/ log(2).
I : valeur du courant injecté exprimé en ampères
U : valeur de la tension mesurée exprimée en volts
e : épaisseur de l'échantillon exprimée en cm

Cette expression ne peut être utilisée que lorsque l'épaisseur du matériau est négligeable devant la distance d existant entre deux pointes (e/d < 0,25). Pour obtenir des épaisseurs suffisamment faibles et ainsi pouvoir calculer la conductivité du matériau, il est préconisé de réaliser la mesure sur un support non conducteur (par exemple, une lame de verre) recouvert du matériau à analyser obtenu par évaporation d'une solution diluée. Afin d'améliorer l'homogénéité du revêtement à analyser, il est également préconisé d'utiliser la technique de dépôt par centrifugation.

Plus particulièrement selon l'invention, on préfère utiliser des polymères conducteurs qui absorbent dans le spectre du visible et en particulier choisis dans le groupe formé par les homopolymères et les copolymères comprenant au moins un motif répétitif choisi parmi:

- les anilines de structure (I) suivante :

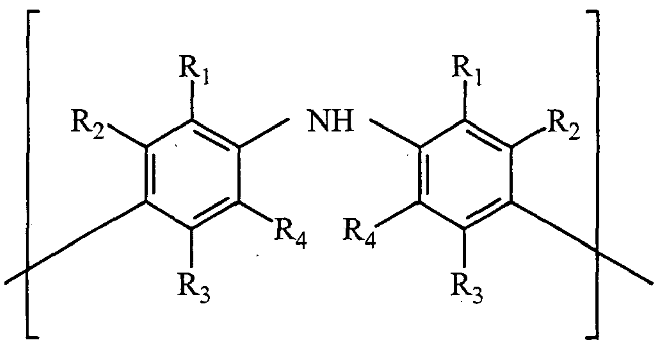

(I)

- les pyrroles de structure (IIa) et (IIb) suivantes :

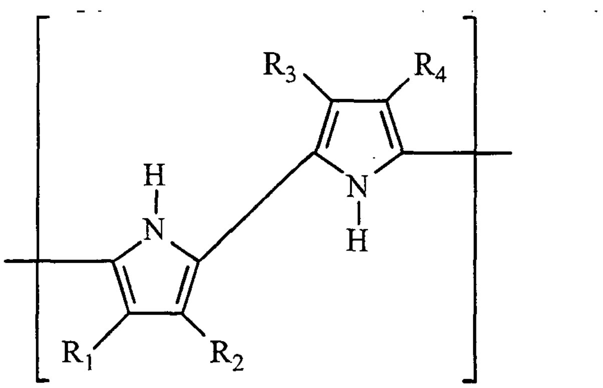

(IIa)

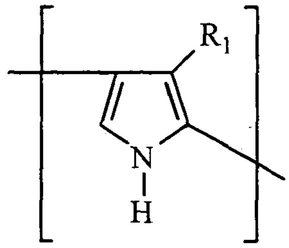

(IIb)

- les thiophènes ou bisthiophènes de formules (IIIa), (IIIb) et (IIIc) suivantes :

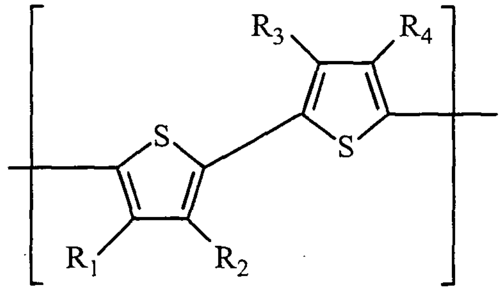

(IIIa)

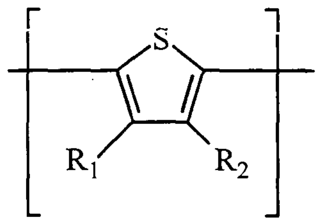

(IIIb)

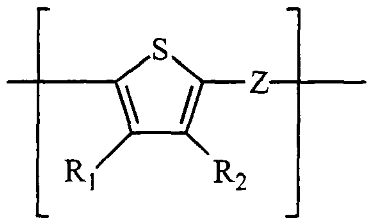

(IIIc)

- les thiophène-vinylènes de formule (III bis) suivante :

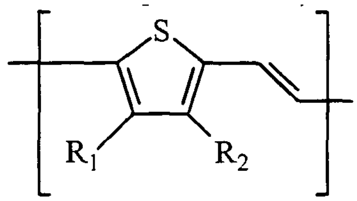

(IIIbis)

- les furanes de formule (IV) suivante :

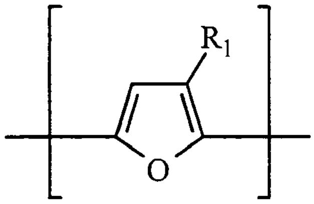

(IV)

- les paraphénylène-sulfures de structure (V) suivante :

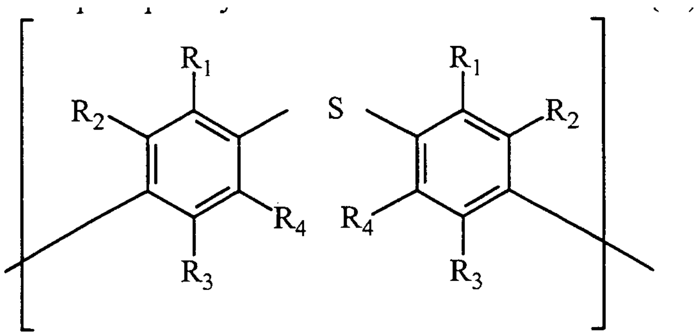

(V)

- les paraphénylène-vinylènes de formule (VI) suivante :

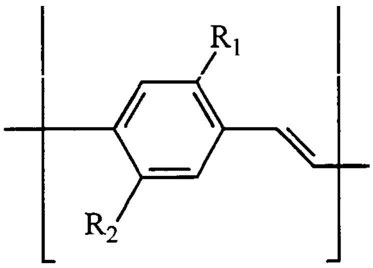

(VI)

- les indoles de formule (VII) suivante :

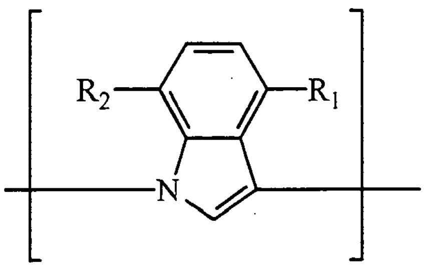

(VII)

- les amides aromatiques de formules (VIIIa), (VIIIb), (VIIIc), (VIIId) suivantes :

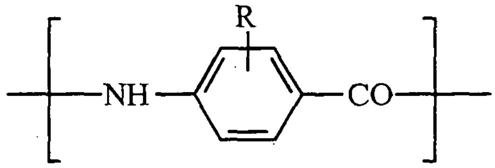

(VIIIa)

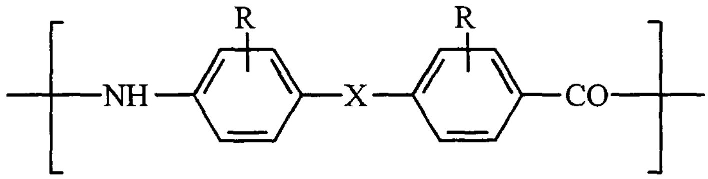

(VIIIb)

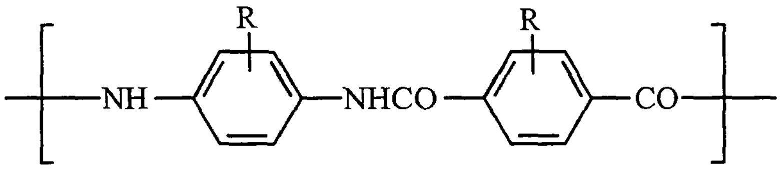

(VIIIc)

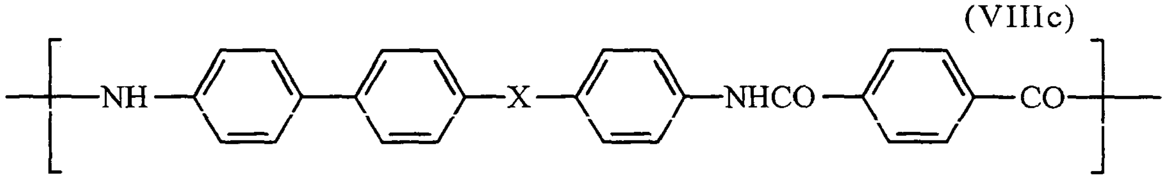

(VIIId)

- les hydrazides aromatiques de formules (IXa), (IXb) et (IXc) suivantes :

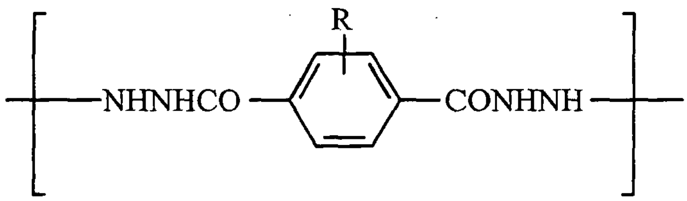

(IXa)

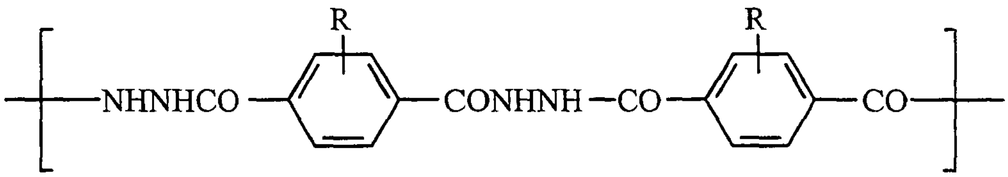

(IXb)

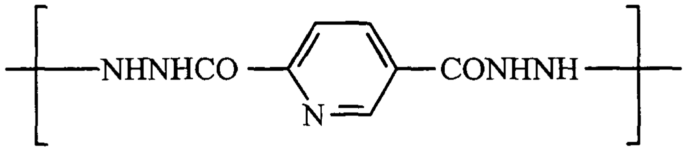

(IXc)

- les azométhines aromatiques de formules (Xa), (Xb) et (Xc) suivantes :

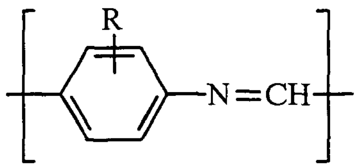

(Xa)

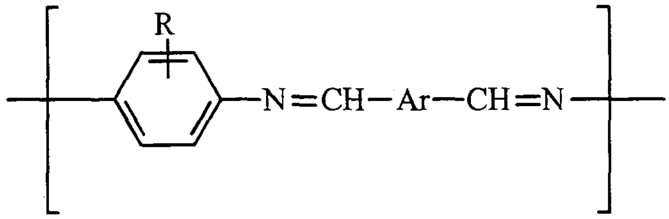

(Xb)

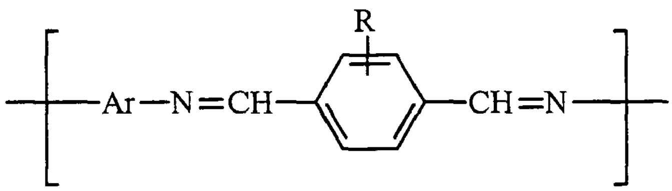

(Xc)

- les esters aromatiques de formules (XIa), (XIb) et (XIc) suivantes :

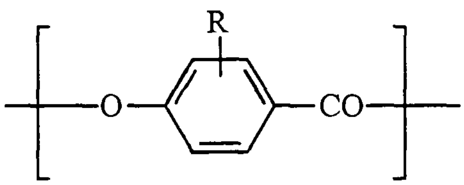

(XIa)

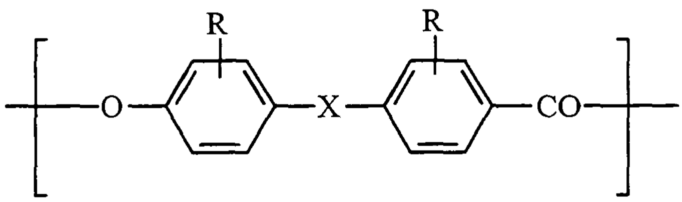

(XIb)

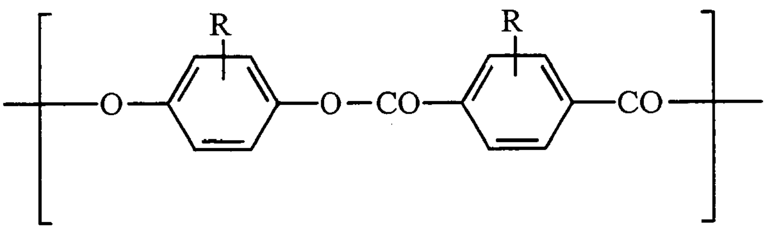

(XIc)

formules (I) à (XI) dans lesquelles :

les radicaux R, $R_1$ à $R_4$, identiques ou différents, sont choisis dans le groupe formé par l'hydrogène, -R', -OR', -COOR', -OCOR', R' représentant un radical alkyle en $C_1$-$C_{20}$, linéaire ou ramifié, un atome d'halogène (choisi de préférence parmi les atomes de chlore, de brome ou d'iode), un radical nitro, un radical cyano, un radical alkylcyano, et des groupements solubilisants ;
X = -NHCO-, -O-, -S-, $-SO_2-$, -N=N-, $-C(CH_3)_2-$, $-CH_2-$, - CH=CH-, -CH=N- ;
Z = -CH=CH- ou -C=C- ; et
Ar représente un radical comprenant un radical monoaromatique ou polyaromatique.

Plus particulièrement, Ar représente au moins un radical choisi parmi les suivants :

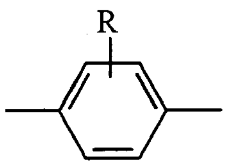 , 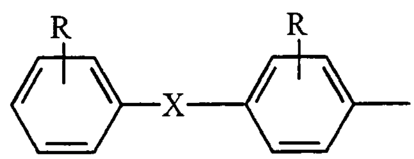 , 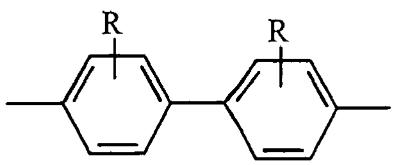 .

De préférence, au moins un des radicaux R et $R_1$ à $R_4$ désigne un groupement solubilisant.
Par groupement solubilisant, on entend au sens de la présente invention, un groupement qui assure la solubilisation de ladite molécule dans le milieu cosmétique, 'de façon que le polymère présente un caractère conducteur après séchage de la composition.
Les groupements solubilisants sont de préférence choisis dans le groupe formé par :

- ■ une fonction acide carboxylique -COOH ou carboxylate $-COO^- M^+$ avec M représentant un métal alcalin comme le sodium ou le potassium, un métal alcalino-terreux, une amine organique telle qu'une amine primaire, secondaire ou tertiaire, une alcanolamine, un acide aminé ; un radical portant une fonction acide carboxylique (-COOH) ou carboxylate ($-COO^- M^+$ avec M tel que défini ci-dessus),
- ■ une fonction acide sulfonique $-SO_3H$ ou sulfonate $-SO_3^- M^+$, M ayant la même définition que ci-dessus, ou un radical portant une fonction acide sulfonique ($-SO_3H$) ou sulfonate ($-SO_3$- $M^+$, M ayant la même définition que ci-dessus),
- ■ un radical amine primaire, secondaire ou tertiaire,
- ■ un radical ammonium quaternaire tel $-NR''_3{}^+ Z'^-$ avec Z'= Br, Cl, alkyl($C_1$-$C_4$)-$OS0_3$ et chaque R'', identique ou différent, désignant un radical alkyle en $C_1$ à $C_{20}$, linéaire ou ramifié, ou formant un hétérocycle avec un autre R'' et l'azote auquel ils sont liés,
- ■ un radical -OH ou un radical hydroxylé tel que hydroxy(alkyl en $C_{1-4}$),
- ■ un radical poly(oxyde d'alcène en $C_2$-$C_3$).

Les fonctions acides carboxyliques ou sulfoniques peuvent être ou ne pas être neutralisées par une base, telle que l'hydroxyde de sodium, le 2-amino-2-méthylpropanol, la triéthylamine ou encore la tributylamine.
Les radicaux amines peuvent être ou ne pas être neutralisés par un acide minéral, tel que l'acide chlorhydrique, ou par un acide organique tel que par exemple, les acides acétique et lactique.
En outre, il est à noter que lesdits radicaux solubilisants peuvent être reliés au cycle par l'intermédiaire d'un groupement espaceur tel que par exemple un radical -R'''-, -OR'''-, -OCOR'''- ou encore -COOR'''-avec R''' représentant un radical alkyle en $C_1$-$C_{20}$, linéaire ou ramifié, comprenant éventuellement un ou plusieurs hétéroatomes, tels que

l'oxygène par exemple.

De préférence les radicaux R, $R_1$ à $R_4$ identiques ou différents, sont choisis parmi l'hydrogène, R', -OR', -OCOR', -COOR' avec R' représentant un radical alkyle en $C_1$-$C_6$, linéaire ou ramifié, et parmi les groupements solubilisants suivants, neutralisés ou non : -COOH, $-CH_2COOH$, $-CH_2OH$, $-(CH_2)_6OH$, $-(CH_2)_3SO_3H$, $-O(CH_2)_3SO_3H$,- O $(CH_2)_3N(CH_2CH_3)_2$, $-[(CH_2)_2O]_xCH_2CH_2OH$, $-[(CH_2)_2O]_XCH_2CH_2OCH_3$ avec x nombre moyen compris entre 0 et 200.

Conformément à un mode de réalisation particulier de l'invention, le polymère conducteur mis en oeuvre comporte au moins un groupement solubilisant par motif répétitif.

Le nombre de motifs répétitifs dans les polymères de l'invention, peut varier de 5 à 10000, notamment de 5 à 1000, plus particulièrement de 10 à 1000 et de préférence de 20 à 700.

Les polymères conducteurs présents dans la composition selon l'invention sont bien connus de l'homme de l'art et décrits notamment dans l'ouvrage "Handbook of organic conductive molécules and polymers" - Wiley 1997- New York, Vol 1, 2, 3, mais aussi dans la revue Can. J. Chem. Vol 64, 1986.

Les polythiophènes et leur synthèse sont plus particulièrement décrits dans l'article tiré de la revue Chem. Mater. 1998, Vol. 10, N°7 pages 1990-1999 - par les auteurs RASMUSSEN S.C., PICKENS J.C. et HUTCHISON J.E., "A new général approach to tuning the properties of functionalized polythiophenes : the oxidative polymerization of monosubstituted bithiophenes" ; dans l'article tiré de la revue Macromolecules 1998, 31, pages 933-936, par les mêmes auteurs "Highly conjugated, water-soluble polymers via direct oxidative polymerization of monosubstituted bithiophenes". Outre la polymérisation par oxydation chimique ou électrochimique, ils peuvent être aussi obtenus par polycondensation (thiophène dihalogéné ; catalyse avec des complexes de nickel ou de palladium) ; par couplage de Suzuki (couplage entre une fonction halogène, brome par exemple, et un acide boronique, catalyse : complexe de palladium et base ; on a alors couplage de type AA-BB (réaction de monomères de type A-X-A avec B-X'-B) ou de type A-B (réaction de plusieurs monomères de type A-X-B)) ; par couplage de Stille (formation de liaison carbone-carbone en présence d'une catalyse à base de Pd- type AA-BB ou A-B), ; par polymérisation de Reike (organozinc en présence d'un complexe de nickel) ; par polymérisation de type McCulloch, etc.

Les polymères conducteurs présents dans la composition selon l'invention sont par ailleurs décrits dans la demande internationale WO 99/47570.

Parmi les polymères conducteurs convenables à la réalisation de la présente invention, on peut citer plus particulièrement les polymères répondant aux formules (IIIa), (IIIb) et (IIIbis) dans lesquelles les groupes solubilisants sont de préférence une fonction acide carboxylique ou un groupement portant une fonction acide carboxylique ; une fonction acide sulfonique ou un groupement portant une fonction acide sulfonique ; un radical amine tertiaire ; un radical ammonium quaternaire tel que $-NR''_3{}^+ Z^-$ avec Z= Br, Cl, alkyl($C_1$-$C_4$)-$OSO_3$ et chaque R" désignant un groupe alkyle en $C_1$ à $C_{20}$, identique ou différent, linéaire ou ramifié, ou formant un hétérocycle avec un autre R" et l'azote auquel ils sont liés ; lesdits groupes étant éventuellement reliés au cycle par un groupement espaceur. Les fonctions acides carboxyliques ou sulfoniques peuvent ou non être neutralisées.

Ainsi, la polymérisation peut être réalisée par oxydation chimique ou électrochimique du monomère thiophène correspondant ou bien encore par polycondensation.

A titre d'illustration, les polythiophènes de formules (IIIa) et (IIIb) peuvent être obtenus par polymérisation par oxydation (par exemple avec une catalyse $FeC1_3$) ; par polycondensation de thiophène dihalogéné catalysée par des complexes de nickel ou de palladium (ex. : $NiCl_2(dppe)_2$) ; par couplage de Suzuki (couplage entre une fonction halogène, brome par exemple et un acide boronique, catalyse : complexe de palladium et base ; on a alors couplage de type AA-BB (réaction de monomères de type A-X-A avec B-X'-B) ou de type A-B (réaction de plusieurs monomères de type A-X-B)); par couplage de Stille (formation de liaison carbone-carbone en présence d'une catalyse à base de Pd- type AA-BB ou A-B) ; par polymérisation de Reike (organozinc en présence d'un complexe de nickel) ; par polymérisation de type McCulloch, etc.

Les polythiophènes vinylènes de formule (IIIc) avec Z représentant -CH=CH-, peuvent notamment être obtenus par polymérisation de Gilch en présence d'une base forte (tertiobutylate de potassium), de 2,5 bis (bromoalkylène) thiophène ; par polymérisation par la méthode de Wessling via l'utilisation de précurseur à base de sels de sulfonium et pyrolyse ; par réaction de Wittig Wittig-Horner.

Les polythiophènes éthynylènes de formule (IIIc) avec Z représentant -C≡C- peuvent être obtenus par couplage de Heck-Sonogashira (de type AA-BB ou A-B ; formation de liaison carbone-carbone entre une fonction acétylénique terminale (ou acétylénique vraie) et une fonction bromo ou iodo catalysée par un complexe de palladium/cuivre (PdC12 (PPh3)3, CuI ou $Cu(Oac)_2$) en présence d'une base telle que la triéthylamine, diisopropylamine, piperidine etc...) ; par méthathèse d'alkynes en présence d'un complexe de molybdène (de $Mo(CO)_6$).

En général la fonctionnalisation des polythiophènes, en d'autres termes, l'apport du ou des groupes solubilisants ou non, est réalisée sur le monomère avant sa polymérisation.

Dans certains cas, l'obtention du groupe solubilisant est obtenu par suite de traitement de polymère. C'est notamment le cas de la fonction acide carboxylique qui peut être obtenue par hydrolyse de l'ester correspondant.

De préférence, le polymère conducteur est choisi parmi ceux de formules (IIIa), (IIIb) et (IIIbis) dans lesquelles

au moins un radical $R_1$ à $R_4$ de la formule (IIIa) ou $R_1$ ou $R_2$ dans les formules (IIIb) ou (IIIbis) représente un groupement solubilisant du type acide carboxylique, sous forme neutralisée ou non, éventuellement relié au cycle par un groupement espaceur, de préférence un radical alkyle en $C_1$-$C_{20}$, linéaire ou ramifié, le ou les autres radicaux représentant un atome d'hydrogène.

Les polymères conducteurs sont généralement présents dans la composition dans des proportions d'au moins 0,001 % en poids, plus particulièrement d'au moins 0,01 % en poids, de préférence d'au moins 0,1 % en poids et de manière encore plus préférée, d'au moins 0,5 % en poids, par rapport au poids total de la composition. Par ailleurs, la teneur en polymère conducteur est avantageusement d'au plus 50 % en poids, plus particulièrement d'au plus 30 % en poids, de préférence d'au plus 20 % en poids et de manière encore plus préférée d'au plus 10 % en poids, par rapport au poids total de la composition.

Selon un mode de réalisation particulièrement avantageux de l'invention, la teneur en polymère conducteur est comprise entre 0,1 et 50% en poids, plus particulièrement entre 0,1 et 30% en poids, et de préférence entre 0,5 et 10 % en poids, par rapport au poids total de la composition.

Par monomère électrophile, on entend un monomère capable de polymériser par polymérisation anionique en présence d'un agent nucléophile tel que par exemple, les ions hydroxydes (OH-) contenus dans l'eau à pH neutre.

Par polymérisation anionique, on entend le mécanisme défini dans l'ouvrage "Advanced Organic Chemistry", Third Edition de Jerry March, pages 151 à 161.

Les monomères électrophiles utilisés plus particulièrement conformément à l'invention sont les monomères de structure :

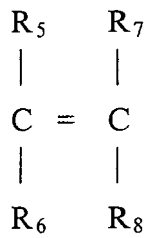

(A)

dans laquelle :

$R_5$ et $R_6$ désignent chacun, indépendamment l'un de l'autre, un groupe peu ou non électro-attracteur (peu ou non inductif-attracteur) tel que :

- un atome d'hydrogène,
- un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi $-OR_5'$, $-COOR_5'$, $-COR_5'$, -SH, $-SR_5'$, -OH, et les atomes d'halogène,
- un résidu polyorganosiloxane modifié ou non,
- un groupement polyoxyalkylène,

$R_7$ et $R_8$ désignent chacun, indépendamment l'un de l'autre, un groupe électro-attracteur (ou inductif-attacteur) choisi de préférence parmi les groupements $-N(R_5')_3^+$, $-S(R_5')_2^+$, $-SH_2^+$, $-NH_3^+$, $-NO_2$, - $SO_2R_5'$, -C≡N, -COOH, $-COOR_5'$, -COSH, $-COSR_5'$, $-CONH_2$, - $CONHR_5'$, -F, -Cl, -Br, -I, $-OR_5'$, $-COR_5'$, -SH, $-SR_5'$, -OH, les groupes alcényle linéaires ou ramifiés, les groupes alcynyle linéaires ou ramifiés, les groupements mono- ou polyfluoroalkyle en $C_1$-$C_4$, les groupements aryle tels que phényle, ou les groupements aryloxy tels que phénoxyloxy,

$R_5'$ désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi $-OR_5''$, - $COOR_5''$, $-COR_5''$, -SH, $-SR_5''$, -OH, les atomes d'halogène et un résidu de polymère pouvant être obtenu par polymérisation radicalaire ou par polycondensation ou par ouverture de cycle, $R_5''$ désignant un groupe alkyle en $C_1$-$C_{10}$.

Par groupement électro-attracteur ou inductif-attracteur (-I), on entend tout groupement plus électronégatif que le carbone. On pourra se reporter à l'ouvrage PR Wells Prog. Phys. Org. Chem., Vol 6,111 (1968).

Par groupement peu ou non électro-attracteur, on entend tout groupement dont l'électronégativité est inférieure ou égale à celle du carbone.

Les groupements alcényle ou alcynyle ont de préférence 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, mieux encore de 1 à 10 atomes de carbone, on peut notamment citer les groupes alkyle, alcényle ou alcynyle linéaires ou ramifiés, tels que méthyle, éthyle, n-butyle, tert-butyle, iso-butyle, pentyle, hexyle, octyle, butényle ou butynyle ; les groupes cycloalkyle ou aromatiques.

Comme groupe hydrocarboné substitué, on peut citer par exemple les groupes hydroxyalkyle ou polyhalogénoalkyle.

A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et/ou siloxy et/ou silanol et/ou amine et/ou imine et/ou fluoroalkyle.

Parmi les groupements polyoxyalkylène, on peut notamment citer les groupements polyoxyéthylène et les groupements polyoxypropylène ayant de préférence 1 à 200 motifs oxyalkylénés.

Parmi les groupements mono- ou polyfluoroalkyle, on peut notamment citer des groupements tels que $-(CH_2)_n-(CF_2)_m-CF_3$ ou $-(CH_2)_n-(CF_2)_m-CHF_2$ avec n=1 à 20 et m= 1 à 20.

Les substituants $R_5$ à $R_8$ peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.

A titre d'exemples de groupement à fonction colorante, on peut notamment citer les groupements azoiques, quinoniques, méthiniques, cyanométhiniques et triarylméthanes.

A titre d'exemples de groupement à fonction antioxydante, on peut notamment citer les groupements de type butylhydroxyanisole (BHA), butylhydroxytoluène (BHT) ou vitamine E.

A titre d'exemples de groupement à fonction filtre UV, on peut notamment citer les groupements de types benzophénones, cinnamates, benzoates, benzylidène-camphres et dibenzoylméthanes.

A titre d'exemples de groupement à fonction conditionnante, on peut notamment citer les groupements cationiques et les groupements de type esters gras.

Parmi les monomères précédemment cités, sont préférés les monomères de la famille des cyanoacrylates et leurs dérivés de formule (B) :

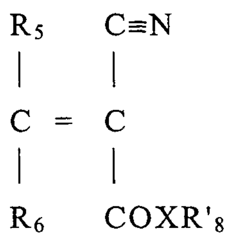

(B)

X désignant NH,S,O,

$R_5$ et $R_6$ ayant les mêmes significations que précédemment,

$R'_8$ pouvant désigner un atome d'hydrogène ou un groupe $R_5'$ tel que défini pour la formule (A).

De préférence, X désigne O.

A titre de composés de formule (B), on peut citer les monomères :

a) appartenant à la famille des 2-cyanoacrylates de polyfluoroalkyle en $C_{1-20}$ tels que :

l'ester 2,2,3,3-tétrafluoropropylique de l'acide 2-cyano-2-propénoïque de formule :

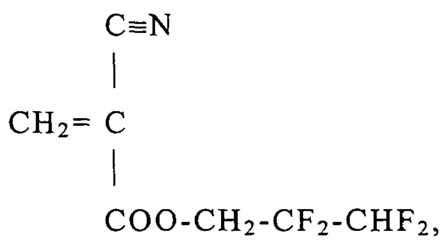

(D)

ou encore l'ester 2,2,2-trifluoroéthylique de l'acide 2-cyano-2-propénoïque de formule :

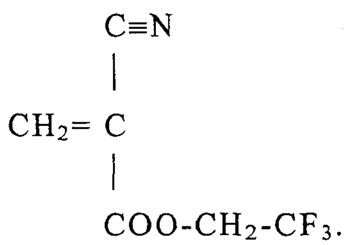

(E)

b) les cyanoacrylates d'alkyle en $C_1$-$C_{10}$ ou d'(alcoxy en $C_{1-}C_4$)(alkyle en $C_1$-$C_{10}$).

On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

Dans le cadre de l'invention, on préfère utiliser les monomères b).

Les monomères le plus particulièrement préférés sont ceux de formule (F) et leurs mélanges :

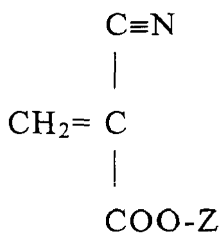

(F)

dans laquelle : $Z=-(CH_2)_7-CH_3$,
$-CH(CH_3)-(CH_2)_5-CH_3$,
$-CH_2-CH(C_2Hs)-(CH_2)_3-CH_3$,
$-(CH_2)_5-CH(CH_3)-CH_3$,
$-(CH_2)_4-CH(C_2H_5)-CH_3$.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Les compositions mises en oeuvre conformément à l'invention ont généralement une concentration en monomère électrophile selon l'invention comprise entre 0,001 et 80 % en poids, et plus particulièrement entre 0,1 et 40 % et encore plus préférentiellement entre 1 et 20 % en poids par rapport au poids total de la composition.

On peut également introduire dans les compositions, des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels

que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfonène et leurs mélanges. Les groupements alkyle désignent de préférence des groupements ayant 1 à 6 atomes de carbone.

On peut aussi utiliser des acides minéraux ou organiques, ces derniers ayant un ou plusieurs groupements carboxyliques ou sulfoniques, présentant un pKa compris entre 0 et 6 tels que l'acide phosphorique, l'acide chlorhydrique, l'acide nitrique, l'acide benzène-ou toluène-sulfonique, l'acide sulfurique, l'acide carbonique, l'acide fluorhydrique, l'acide acétique, l'acide formique, l'acide propionique, l'acide benzoïque, les acides mono-, di- ou trichloroacétiques, l'acide salicylique et l'acide trifluoroacétique.

La quantité d'inhibiteur peut aller de 10 ppm à 20%, et plus préférentiellement de 10 ppm à 5% et encore plus préférentiellement de 10 ppm à 1 % en poids par rapport au poids total de la composition.

Par milieu cosmétiquement acceptable, on entend un milieu compatible avec les fibres kératiniques telles que les cheveux.

Le milieu cosmétiquement acceptable est de préférence anhydre. On entend par « milieu anhydre », un milieu contenant moins de 1 % en poids d'eau par rapport au poids total de la composition.

Le milieu cosmétiquement acceptable est de préférence choisi parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ; les cires ; ou encore des composés organiques tels que des alcanes en $C_5$-$C_{10}$, l'acétone, la méthyléthylcétone, les esters d'acides en $C_1$-$C_{20}$ et d'alcools en $C_1$-$C_8$ tels que l'acétate de méthyle, l'acétate de butyle, l'acétate d'éthyle et le myristate d'isopropyle, le diméthoxyéthane, le diéthoxyéthane, les alcools gras en $C_{10}$-$C_{30}$ tels que l'alcool laurique, l'alcool cétylique, l'alcool stéarylique et l'alcool béhénylique, les acides gras en $C_{10}$-$C_{30}$ tels que l'acide laurique et l'acide stéarique, les amides gras en $C_{10}$-$C_{30}$ tels que le diéthanolamide laurique, les esters d'alcools gras en $C_{10}$-$C_{30}$ tels que les benzoates d'alcool gras en $C_{10}$-$C_{30}$ et leurs mélanges.

De préférence, les composés organiques sont choisis parmi les composés liquides à la température de 25°C et sous $10^5$ Pa (760mm de Hg).

Les compositions conformes à l'invention peuvent également contenir au moins un agent utilisé habituellement en cosmétique, choisi, par exemple, parmi des agents réducteurs, des corps gras, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des tensio-actifs anioniques, non ioniques ou amphotères, des polymères fixants ou non, des polyols, des protéines, des vitamines, des colorants directs ou d'oxydation, des agents nacrants, des propulseurs, et des épaississants minéraux ou organiques tels que le benzylidène-sorbitol et les N-acylaminoacides.

Ces agents peuvent être éventuellement encapsulés. La capsule peut être de type polycyanoacrylate.

Le propulseur est notamment utilisé pour la préparation de compositions aérosol. Il comprend généralement des gaz comprimés et/ou liquéfiés bien connus dans la technique. De préférence, on utilise l'air, le gaz carbonique, l'azote comprimé ou encore un gaz comme le diméthyléther, les hydrocarbures halogénés, par exemple fluorés, ou non, ou leur mélange.

La composition est utilisée sur les fibres kératiniques, et plus particulièrement sur les cheveux, de préférence en présence d'un agent nocléophile, pour leur traitement cosmétique.

Le procédé de traitement cosmétique selon l'invention comprend l'application d'une composition telle que définie ci-dessus, sur les fibres kératiniques, et plus particulièrement sur les cheveux, en présence d'un agent nucléophile tel que défini ci-dessous.

Les agents nucléophiles susceptibles d'initier la polymérisation anionique sont des systèmes connus en eux-mêmes, capables de générer un carbanion au contact d'un agent nucléophile, tels que les ions hydroxydes contenus dans l'eau à pH neutre. On entend par « carbanion », les espèces chimiques définies dans « Advanced Organic Chemistry, Third Edition », de Jerry March, page 141.

Les agents nucléophiles peuvent être constitués par un composé moléculaire, un oligomère, un dendrimère ou un polymère possédant des fonctions nucléophiles. De façon non limitative, on peut citer comme fonctions nucléophiles les fonctions : $R^{iv}{}_2N^-$, $NH_2^-$, $Ph_3C^-$, $R^{iv}{}_3C^-$, $PhNH^-$, pyridine, $ArS^-$, $R^{iv}$-C≡$C^-$, $R^{iv}S^-$, SH, $R^{iv}O^-$, $R^{iv}{}_2NH$, $ArO^-$, $N_3^-$, $OH^-$, $ArNH_2$, $NH_3$, $I^-$, $Br^-$, $Cl^-$, $R^{iv}COO^-$, $SCN^-$, $R^{iv}OH$, $R^{iv}SH$, $NCO^-$, $CN^-$, $NO_3^-$, $ClO_4^-$ et $H_2O$, Ph représentant le groupe phényle ; Ar représentant un groupe aryle et $R^{iv}$ représentant un groupe alkyle en $C_1$-$C_{10}$.

De préférence, l'agent nucléophile est l'eau. Cette eau peut être apportée par une humidification préalable.

Il est également possible, afin de moduler la cinétique de réaction, de préalablement humidifier les fibres kératiniques comme les cheveux, à l'aide d'une solution aqueuse dont le pH a été ajusté à l'aide d'une base, d'un acide ou d'un mélange acide/base. L'acide et/ou la base peuvent être inorganiques ou organiques.

Il est également possible de moduler la cinétique de polymérisation par voie anionique en pré-imprégnant les fibres kératiniques comme les cheveux, à l'aide d'un agent nucléophile autre que l'eau. L'agent nucléophile peut être utilisé pur, en solution, sous forme d'une émulsion ou être encapsulé.

Pour moduler la cinétique de polymérisation anionique, on peut également augmenter la nucléophilie des fibres kératiniques comme les cheveux, par transformation chimique de la fibre kératinique.

A titre d'exemple de transformation chimique, on peut citer la réduction des ponts di-sulfure composant en partie la kératine en thiols avant application de la composition de l'invention. De façon non exhaustive, on peut citer comme réducteurs des ponts di-sulfure composant en partie la kératine, les composés suivants:

- thiosulfate de sodium anhydre,
- métabisulfite de sodium en poudre,
- thiourée,
- sulfite d'ammonium,
- acide thioglycolique,
- acide thiolactique,
- thiolactate d'ammonium,
- mono-thioglycolate de glycérol,
- thioglycolate d'ammonium,
- thioglycérol,
- acide 2,5-dihydroxybenzoique,
- di-thioglycolate de diammonium,
- thioglycolate de strontium,
- thioglycolate de calcium,
- formo-sulfoxylate de zinc,
- thioglycolate d'isooctyle,
- dl-cystéine,
- thioglycolate de monoéthanolamine.

Pour moduler la cinétique de polymérisation par voie anionique, et plus précisément réduire la vitesse de polymérisation des monomères de l'invention, il est possible d'augmenter la viscosité de la composition. Pour ce faire, on peut ajouter à la composition de l'invention un ou des polymères ne présentant pas de réactivité sur les monomères conformes à l'invention. Dans ce cadre, on peut citer de façon non exhaustive le poly(méthacrylate de méthyle) (PMMA) ou encore les copolymères à base de cyanoacrylate tels qu'ils sont décrits dans le brevet US 6,224,622.

Afin d'améliorer entre autres l'adhésion du poly(cyanoacrylate) formé in situ, on peut pré-traiter la fibre avec tous types de polymères, ou réaliser un traitement capillaire avant application de la composition de l'invention, comme une coloration directe ou d'oxydation, une permanente ou encore un défrisage.

L'application des compositions telles que décrites ci-dessus peut être suivie ou non d'un rinçage.

Elles peuvent se présenter sous la forme de lotion, de spray, de mousse, et être appliquées comme shampoing ou après-shampoing.

Un autre objet de l'invention consiste en un procédé de traitement cosmétique des fibres kératiniques, comprenant au moins deux étapes, une étape comprenant l'application d'au moins un polymère conducteur tel que défini ci-dessus, et, avec ou sans rinçage intermédiaire, une autre étape comprenant l'application d'au moins un monomère électrophile tel que défini ci-dessus, l'ordre des étapes étant indifférent.

Un mode de réalisation particulier de l'invention consiste en ce que l'application du polymère conducteur se fait avant l'application d'au moins un monomère électrophile.

Un autre mode de réalisation particulier de l'invention consiste en ce que l'application d'au moins un monomère électrophile se fait avant l'application d'au moins un polymère conducteur.

Un mode de réalisation particulièrement préféré de l'invention consiste en un procédé comprenant les étapes consistant à ::

(1) appliquer sur les cheveux une composition comprenant au moins un polymère conducteur,
(2) appliquer sur les cheveux, après un éventuel rinçage intermédiaire, au moins un monomère électrophile.

L'ordre des étapes (1) et (2) peut être inversé.

Un autre objet de l'invention consiste en un kit comprenant une première composition contenant au moins un monomère électrophile et éventuellement au moins un inhibiteur de polymérisation anionique et/ou radicalaire, ainsi qu'une deuxième composition comprenant dans un milieu cosmétiquement acceptable au moins un polymère conducteur solubic dans ledit milieu, tel que défini ci-dessus.

Les exemples suivants sont donnés à titre illustratif de la présente invention.

Dans les exemples suivants, toutes les quantités sont indiquées en pour cent en poids de matière active par rapport au poids total de la composition, sauf indication contraire.

EXEMPLES

Exemple 1 : Préparation du poly(thiophène-3- acide acétique)

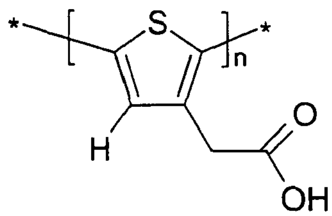

1) Préparation du polymère : poly(thiophène-3-acétate d'éthyle)

Dans un tube de Schlenk sous argon, on introduit 25 ml de chloroforme sec, on dégaze puis on introduit les réactifs suivants :

- 2,5 g de thiophène-3-acétate d'éthyle (14,7 mmol)

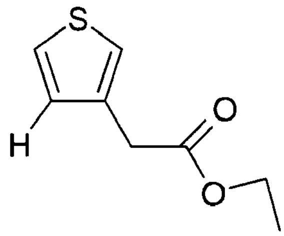

et

- 1 g de $FeCl_3$ (6,15 mmol).

Le mélange est agité pendant 24 heures sous argon à 50°C.
Le polymère poly(thiophène-3-acétate d'éthyle) est alors précipité dans l'heptane, puis dissous dans une solution de tétrahydrofuranne.

Caractérisation par spectrométrie infra rouge :

Bande du C=O : 1719 $cm^{-1}$ ; bandes du $CH_2$, $CH_3$ = 2979 $cm^{-1}$ , 2934 $cm^{-1}$ et disparition de la bande CH à 3102 $cm^{-1}$ présente dans le monomère.

2) Hydrolyse du polymère poly(thiophène-3-acétate d'éthyle) pour former le poly(thiophène-3- acide acétique).

On effectue une hydrolyse du polymère obtenu précédemment avec un excès de 50 ml d'une solution aqueuse d'hydroxyde de sodium (2N) pendant 48 heures à 70°C, suivie d'une acidification par HC1 concentré jusqu'à précipitation du produit poly(thiophèrle-3-acide acétique).

Le polymère est ensuite filtré et lavé à plusieurs reprises par de l'eau distillée afin d'éliminer les traces de catalyseur.

Caractérisation du polymère par spectrométrie infra-rouge:

Bande du C=O : 1740 $cm^{-1}$ ; COO 1580 $cm^{-1}$ ; OH (bande large 3000-3500 $cm^{-1}$)

3) Neutralisation du polymère poly(thiophène-3- acide acétique) :

Le polymère poly(thiophène-3- acide acétique) (2g) est dissous dans le tétrahydrofuranne (30g) et neutralisé à raison de 1 mol d'hydroxyde de sodium par mole d'acide carboxylique. L'eau (30 g) est ensuite additionnée. Le tétrahydrofuranne est évaporé.

Il est ainsi obtenu une solution aqueuse 6% de poly(thiophène-3- acide acétique) sous forme d'un sel de sodium.

Exemple 2

On prépare la composition A à partir des ingrédients suivants, les proportions étant indiquées en pour cent en poids par rapport au poids total de la composition :

| | |
|---|---|
| - 2-octylcyanoacrylate stabilisé avec 1% d'acide phosphorique | 10 % |
| - Mélange 50/50 en poids de DC 1501 Fluid (Dow Corning) (poly(alpha-omega dihydroxyl- diméthylsiloxane/ cyclopentadiméthylsiloxane) (14,7/85,3)) et de DC 245 Fluid (Dow Corning) (cyclopentadiméthylsiloxane) | 90 % |

On prépare la composition B à partir des ingrédients suivants, les proportions étant indiquées en pour cent en poids par rapport au poids total de la composition :

| | | |
|---|---|---|
| Poly(thiophène-3-acide acétique)obtenu ci-dessus | | 5 |
| Aminométhyl propanol | qs | pH 7 |
| Alcool éthylique | | 15 |
| Eau | qs | 100 |

On applique sur des mèches de 1 g. de cheveux châtain naturels à température ambiante (20 °C), 0,1 g de la composition B décrite ci-dessus. Après 20 minutes de pose, on laisse sécher la mèche ainsi traitée.

La mèche obtenue est humidifiée avec de l'eau puis 0,5 g de composition A est appliqué. Après 15 minutes de pose, la mèche est séchée au sèche-cheveux.

Les mèches obtenues présentent un aspect très brillant. Au toucher, les cheveux présentent l'avantage de ne pas être perçus comme gras, les propriétés optiques observées se conservent avec les shampooings.

Exemple 3

On prépare la composition C à partir des ingrédients suivants, les proportions étant indiquées en pour cent en poids par rapport au poids total de la composition:

| | |
|---|---|
| Cyanoacrylate de méthylheptyle* | 10% |
| Mélange 50/50 en poids de DC 1501 Fluid (Dow Corning) (poly(alpha-omega dihydroxyl- diméthylsiloxane/ cyclopentadiméthylsiloxane) (14,7/85,3)) et de DC 245 Fluid (Dow Corning) (cyclopentadiméthylsiloxane) | 90 % |
| * commercialisé par la société Chemence, UK | |

On prépare la composition D à partir des ingrédients suivants, les proportions étant indiquées en pour cent en poids par rapport au poids total de la composition :

| | |
|---|---|
| Baytron P* | 80 |

(suite)

| | |
|---|---|
| Eau qs | 100 |
| * dispersion aqueuse de polyéthylène dioxythiophène/ polystyrène sulfonate commercialisée par la société BAYER, avec un extrait sec de 1,3%. | |

On applique sur des mèches de 1 g. de cheveux châtain naturels à température ambiante (20 °C), 0,1 g de la composition D décrite ci-dessus. Après 20 minutes de pose, on laisse sécher la mèche ainsi traitée.

La mèche obtenue est humidifiée avec de l'eau puis 0,5 g de composition C sont appliqués. Après 15 minutes de pose, la mèche est séchée au sèche-cheveux.

Les mèches obtenues présentent un aspect brillant avec une sensation d'enrobage. Au toucher, les cheveux présentent l'avantage de ne pas être perçus comme gras. Les propriétés optiques et la sensation d'enrobage observées se conservent avec les shampooings.

Exemple 4

On prépare la composition E à partir des ingrédients suivants, les proportions étant indiquées en pour cent en poids par rapport au poids total de la composition :

| | |
|---|---|
| Cyanoacrylate de méthylhentyle* | 10 % |
| Acide acétique | 0,25% |
| Mélange 50/50 en poids de DC 1501 Fluid (Dow Corning) (poly(alpha-omega dihydroxyl- diméthylsiloxane/ cyclopentadiméthylsiloxane) (14,7/85,3)) et de DC 245 Fluid (Dow Corning) (cyclopentadiméthylsiloxane) | 89,75% |
| * commercialisé par la société Chemence, UK | |

On prépare la composition D à partir des ingrédients suivants, les proportions étant indiquées en pour cent en poids par rapport au poids total de la composition :

| | |
|---|---|
| Baytron P* | 80 |
| Eau qs | 100 |
| * dispersion aqueuse de polyéthylène dioxythiophène/ polystyrène sulfonate commercialisée par la société BAYER, avec un extrait sec de 1,3%. | |

On applique sur des mèches de 1 g. de cheveux châtain naturels à température ambiante (20 °C), 0,1 g de la composition D décrite ci-dessus. Après 20 minutes de pose, on laisse sécher la mèche ainsi traitée.

La mèche obtenue est humidifiée avec de l'eau puis 0,5 g de composition E sont appliqués. Après 15 minutes de pose, la mèche est séchée au sèche-cheveux.

Les mèches obtenues présentent un aspect brillant avec une sensation d'enrobage. Au toucher, les cheveux présentent l'avantage de ne pas être perçus comme gras. Les propriétés optiques et la sensation d'enrobage observées se conservent avec les shampooings

Exemple 5

On prépare la composition F à partir des ingrédients suivants, les proportions étant indiquées en pour cent en poids par rapport au poids total de la compositon :

| | |
|---|---|
| Cyanoacrylate de méthylheptyle* | 10 % |
| Acide acétique | 0,25% |
| Nacre mica enrobé d'oxyde de fer brun commercialisée par Eckart, sous le nom Prestige Bronze | 10% |
| Mélange 50/50 en poids de DC 1501 Fluid (Dow Corning) (poly(alpha-omega dihydroxyl-diméthylsiloxane/ cyclopentadiméthylsiloxane) (14,7/85,3)) et de DC 245 Fluid (Dow Corning) (cyclopentadiméthylsiloxane) | 79,75 % |
| * commercialisé par la société Chemence, UK | |

On prépare la composition D à partir des ingrédients suivants, les proportions étant indiquées en pour cent en poids par rapport au poids total de la composition :

| | |
|---|---|
| Baytron P* | 80 |
| Eau qs | 100 |
| * dispersion aqueuse de polyethylene dioxythiophène/ polystyrene sulfonate commercialisée par la société BAYER, avec un extrait sec de 1,3%. | |

On applique sur des mèches de 1 g. de cheveux châtain naturels à température ambiante (20 °C), 0,1 g de la composition D décrite ci-dessus. Après 20 minutes de pose, on laisse sécher la mèche ainsi traitée.

La mèche obtenue est humidifiée avec de l'eau puis 0,5 g de composition F sont appliqués. Après 15 minutes de pose, la mèche est séchée au sèche-cheveux.

Les mèches obtenues sont colorées et présentent un aspect brillant avec une sensation d'enrobage. Au toucher, les cheveux présentent l'avantage de ne pas être perçus comme gras. Les propriétés optiques et la sensation d'enrobage observées, ainsi que la couleur des mèches, se conservent avec les shampooings.

Exemple 6

On prépare la composition G à partir des ingrédients suivants, les proportions étant indiquées en pour cent en poids par rapport au poids total de la composition :

| | |
|---|---|
| Cyanoacrylate d'éthylhexyle O-60* | 10 % |
| Acide acétique | 0.25% |
| Mélange 50/50 en poids de DC 1501 Fluid (Dow Corning) (poly(alpha-omega dihydroxyl- diméthylsiloxane/ cyclopentadiméthylsiloxane) (14,7/85,3)) et de DC 245 Fluid (Dow Corning) (cyclopentadiméthylsiloxane) | 89.75% |
| * commercialisé par la société Tong Shen, Chine | |

On prépare la composition D à partir des ingrédients suivants, les proportions étant indiquées en pour cent en poids par rapport au poids total de la composition :

| | |
|---|---|
| Baytron P* | 80 |

(suite)

| | |
|---|---|
| Eau qs | 100 |
| * dispersion aqueuse de polyéthylene dioxythiophène/ polystyrène sulfonate commercialisée par la société BAYER, avec un extrait sec de 1,3%. | |

On applique sur des mèches de 1 g. de cheveux châtain naturels à température ambiante (20 °C), 0,1 g de la composition D décrite ci-dessus. Après 20 minutes de pose, on laisse sécher la mèche ainsi traitée.

La mèche obtenue est humidifiée avec de l'eau puis 0,5 g de composition G sont appliqués. Après 15 minutes de pose, la mèche est séchée au sèche-cheveux.

Les mèches obtenues présentent un aspect brillant avec une sensation d'enrobage. Au toucher, les cheveux présentent l'avantage de ne pas être perçus comme gras. Les propriétés optiques et la sensation d'enrobage observées se conservent avec les shampooings.

## Revendications

1. Composition comprenant dans un milieu cosmétiquement acceptable, au moins un monomère électrophile, et au moins un polymère conducteur.

2. Composition selon la revendication 1, **caractérisée en ce que** le polymère conducteur est choisi parmi les homopolymères et les copolymères comprenant au moins un motif répétitif choisi parmi :

   - les anilines de structure (I) suivante :

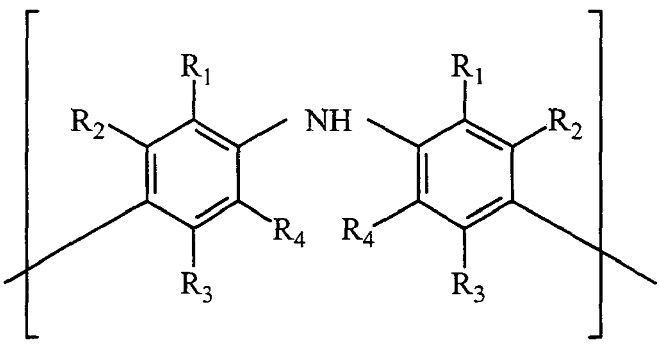

(I)

   - les pyrroles de structure (IIa) et (IIb) suivantes :

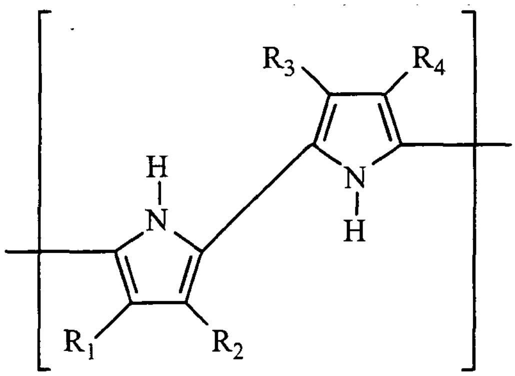

(IIa)

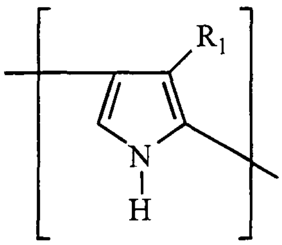

(IIb)

- les thiophènes ou bisthiophènes de formules (IIIa), (IIIb) et (IIIc) suivantes :

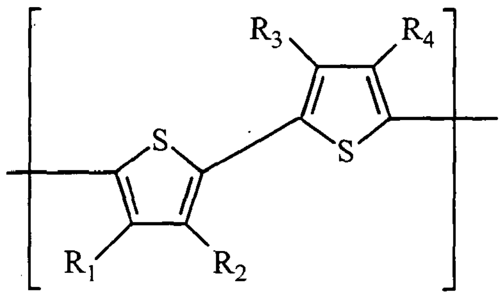

(IIIa)

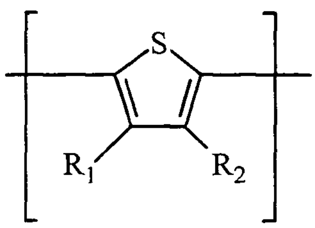

(IIIb)

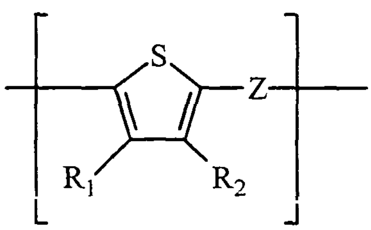

(IIIc)

- les thiophène-vinylènes de formule (III bis) suivante :

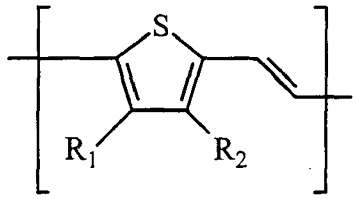

(IIIbis)

- les furanes de formule (IV) suivante :

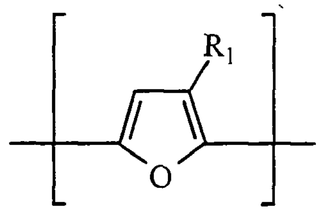

(IV)

- les paraphénylène-sulfures de structure (V) suivante :

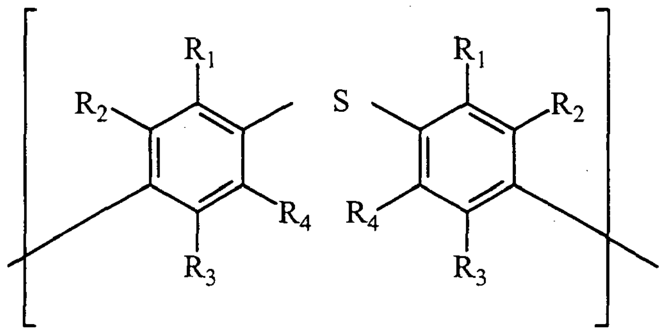

(V)

- les paraphénylène-vinylènes de formule (VI) suivante :

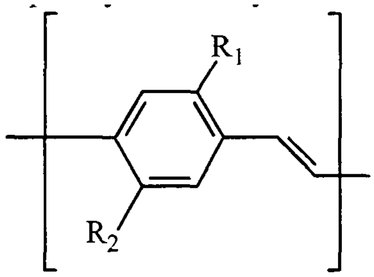

(VI)

- les indoles de formule (VII) suivante :

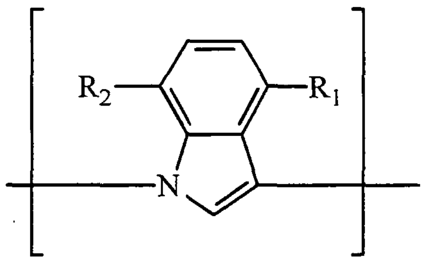

(VII)

- les amides aromatiques de formules (VIIIa), (VIIIb), (VIIIc), (VIIId) suivantes :

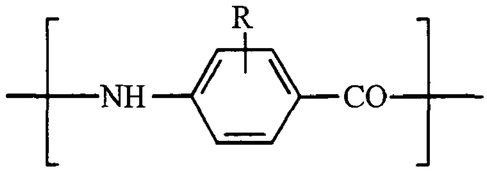

(VIIIa)

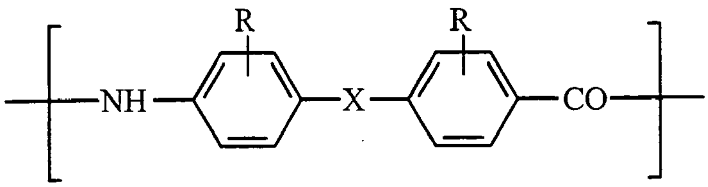

(VIIIb)

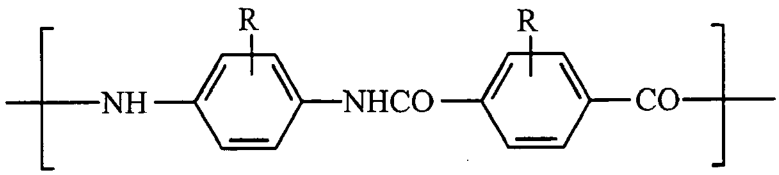

(VIIIc)

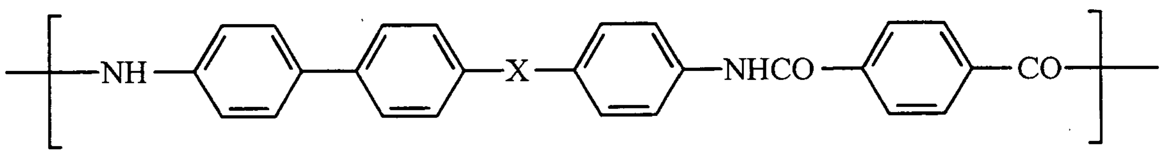

(VIIId)

- les hydrazides aromatiques de formules (IXa), (IXb) et (IXc) suivantes :

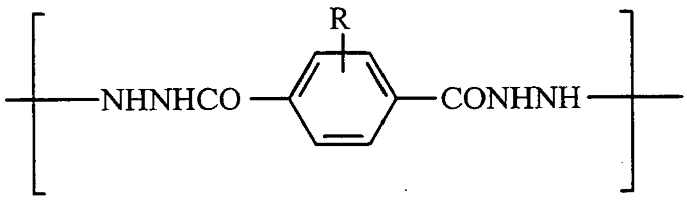

(IXa)

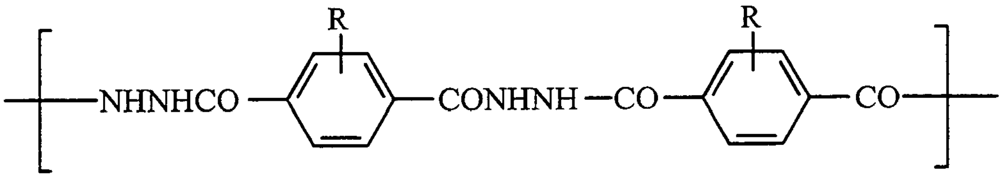

(IXb)

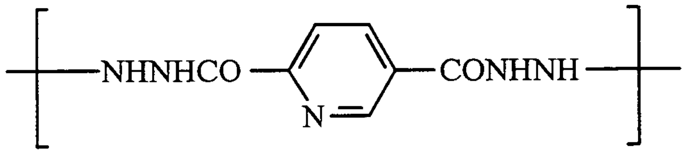

(IXc)

- les azométhines aromatiques de formules (Xa), (Xb) et (Xc) suivantes :

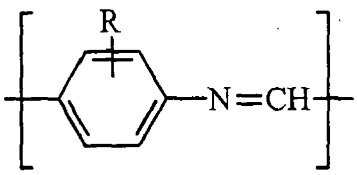

(Xa)

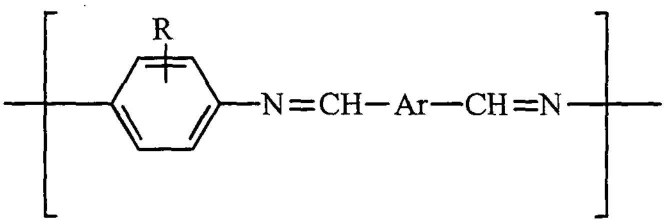

(Xb)

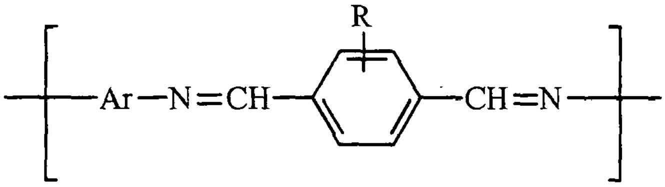

(Xc)

- les esters aromatiques de formules (XIa), (XIb) et (XIc) suivantes :

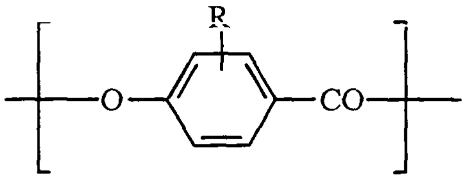

(XIa)

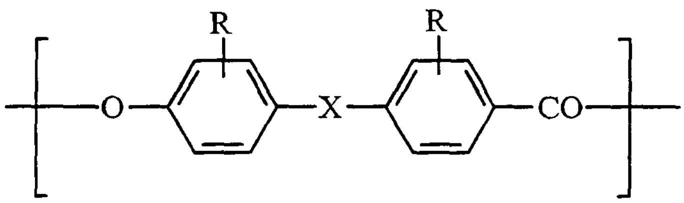

(XIb)

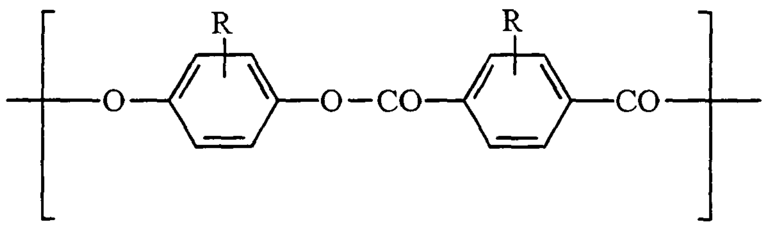

(XIc)

formules (I) à (XI) dans lesquelles :

les radicaux R, $R_1$ à $R_4$, identiques ou différents, sont choisis dans le groupe formé par l'hydrogène, -R', -OR', -COOR', -OCOR', R' représentant un radical alkyle en $C_1$-$C_{20}$, linéaire ou ramifié, un atome d'halogène, un radical nitro, un radical cyano, un radical alkylcyano, et des groupements solubilisants ;
X = -NHCO-, -O-, -S-, $-SO_2-$, -N=N-, $-C(CH_3)_2-$, $-CH_2-$, -CH=CH-, - CH=N-;
Z = -CH=CH- ou -C≡C- ; et
Ar représente un radical comprenant un radical monoaromatique ou polyaromatique.

**3.** Composition selon la revendication 2, **caractérisée en ce qu'**au moins des groupes R et $R_1$ à $R_4$ désigne un groupement solubilisant.

**4.** Composition selon la revendication 2 ou 3, **caractérisée en ce que** les groupements solubilisants sont choisis dans le groupe formé par :

■ une fonction acide carboxylique -COOH ou carboxylate $-COO^-M^+$ avec M représentant un métal alcalin, un métal alcalino-terreux, une amine organique telle qu'une amine primaire, secondaire ou tertiaire, une alcanolamine, un acide aminé ; un radical portant une fonction acide carboxylique ou carboxylate,
■ une fonction acide sulfonique $-SO_3H$ ou sulfonate $-SO_3^-$ $M^+$, M ayant la même définition que ci-dessus, ou un radical portant une fonction acide sulfonique ou sulfonate,
■ un radical amine primaire, secondaire ou tertiaire,
■ un radical ammonium quaternaire tel $-NR''_3{}^+$ $Z'^-$ avec Z'= Br, Cl, alkyl($C_1$-$C_4$)-$OSO_3$ et chaque R" identique ou différent, désignant un radical alkyle en $C_1$ à $C_{20}$, linéaire ou ramifié, ou formant un hétérocycle avec un autre R" et l'azote auquel ils sont liés,
■ un radical -OH ou un radical hydroxylé,
■ un radical poly(oxyde d'alcène en $C_2$-$C_3$).

**5.** Composition selon l'une quelconque des revendications précédentes, **caractérisée en ce que** le polymère comporte au moins un groupement solubilisant par motif répétitif.

**6.** Composition selon l'une des revendications précédentes, **caractérisé en ce que** le polymère conducteur répond aux formules (IIIa), (IIIb) ou (IIIbis), dans lesquelles au moins un radical $R_1$ à $R_4$ de la formule (IIIa) ou $R_1$ ou $R_2$ des formules (IIIb) ou (IIIbis) représente un groupement solubilisant du type acide carboxylique, sous forme neutralisée ou non, éventuellement relié au cycle par un groupement espaceur, de préférence un radical alkyle en $C_1$-$C_{20}$, linéaire ou ramifié, le ou les autres radicaux représentant un atome d'hydrogène.

**7.** Composition selon l'une quelconque des revendications précédentes, **caractérisée en ce que** le polymère conducteur est soluble dans le milieu cosmétiquement acceptable.

**8.** Composition selon l'une quelconque des revendications précédentes, **caractérisée en ce que** le ou les polymères conducteurs sont présents dans des proportions d'au moins 0,001 % en poids, de préférence d'au moins 0,01 % en poids par rapport au poids total de la composition.

**9.** Composition selon l'une quelconque des revendications précédentes, **caractérisée en ce que** le ou les polymères conducteurs sont présents dans des proportions d'au plus 50 % en poids, de préférence d'au plus 30 % en poids par rapport au poids total de la composition.

**10.** Composition selon l'une quelconque des revendications précédentes, **caractérisée en ce que** le ou les monomères électrophiles répondent à la formule :

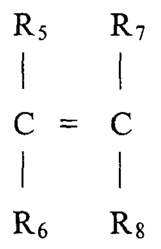

(A)

dans laquelle :

$R_5$ et $R_6$ désignent chacun, indépendamment l'un de l'autre, un groupe peu ou non électro-attracteur choisi parmi :

- un atome d'hydrogène,
- un groupe hydrocarboné saturé ou non, linéaire, ramifié ou cyclique, comportant de 1 à 20 atomes de carbone et contenant éventuellement un ou plusieurs atomes d'azote, d'oxygène, de soufre, et éventuellement substitué par un ou plusieurs groupements choisi parmi $-OR_5'$, $-COOR_5'$, $-COR_5'$, -SH, $-SR_5'$, -OH et les atomes d'halogène,
- un résidu polyorganosiloxane modifié ou non,
- un groupement polyoxyalkylène,

$R_7$ et $R_8$ désignent chacun, indépendamment l'un de l'autre, un groupe électro-attracteur choisi parmi les groupements $-N(R_5')3+1$ - $-S(R_5')_2^+$, $-SH_2^+$, $-NH_3^+$, $-NO_2$, $-SO_2R_5'$ -C≡N, -COOH, $-COOR_5'$, -COSH, $-COSR_5'$, $-CONH_2$, $-CONHR_5'$, -F, -Cl, -Br, -1, $-OR_5'$, $-COR_5'$, -SH, $-SR_5'$, -OH, les groupes alcényle linéaires ou ramifiés, les groupes alcynyle linéaires ou ramifiés, les groupements mono- ou polyfluoroalkyle en $C_1$-$C_4$, les groupements aryle et aryloxy.

$R_5'$ désigne un groupe hydrocarboné saturé ou non, linéaire, ramifié ou cyclique, comportant de 1 à 20 atomes de carbone et contenant éventuellement un ou plusieurs atomes d'azote, d'oxygène, de soufre, et éventuellement substitué par un ou plusieurs groupements choisi parmi $-OR_5''$, $-COOR_5''$, $-COR_5''$, -SH, $-SR_5''$, -OH, les atomes d'halogène, et un résidu de polymère pouvant être obtenu par polymérisation radicalaire ou par polycondensation ou par ouverture de cycle, $R_5''$ désignant un groupe alkyle en $C_1$-$C_{10}$.

**11.** Composition selon la revendication 10, **caractérisée en ce que** le ou les monomères électrophiles sont choisis parmi les composés de formule :

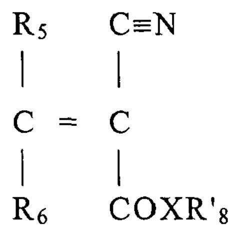

(B)

X désignant NH,S,O,
$R_5$ et $R_6$ sont tels que définis dans la revendication 10,
$R'_8$ pouvant désigner un atome d'hydrogène ou un groupe $R_5'$ tel que défini dans la revendication 10.

**12.** Composition selon la revendication 11, **caractérisée par le fait que** le ou les monomères sont choisis parmi les 2-cyanoacrylates de polyfluoroalkyle en $C_{1-20}$, les cyanoacrylates d'alkyle en ($C_1$-$C_{10}$) ou d'(alcoxy en $C_1$-$C_4$)(alkyle en $C_1$-$C_{10}$).

**13.** Composition selon la revendication 12, **caractérisée en ce que** le ou les monomères sont choisis parmi le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

**14.** Composition selon la revendication 13, **caractérisée en ce que** le ou les monomères électrophiles répondent à la formule (F) :

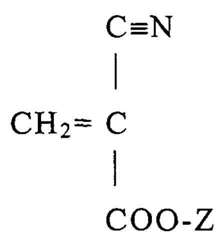

(F)

dans laquelle : $Z=-(CH_2)_7-CH_3$,
$-CH(CH_3)-(CH_2)_5-CH_3$,
$-CH_2-CH(C_2H_5)-(CH_2)_3-CH_3$,
$-(CH_2)_5-CH(CH_3)-CH_3$,
$-(CH_2)_4-CH(C_2H_5)-CH_3$.

**15.** Composition selon l'une quelconque des revendications précédentes, **caractérisée en ce que** le ou les monomères électrophiles sont fixés de façon covalente sur des supports tels que des polymères, des oligomères ou des dendrimères.

**16.** Composition selon l'une quelconque des revendications précédentes, **caractérisée en ce que** la quantité de monomère électrophile va de 0,001 à 80 % en poids, de préférence de 0,1 à 40 % en poids par rapport au poids total de la composition.

**17.** Composition selon l'une quelconque des revendications précédentes, **caractérisée en ce que** le milieu cosmétiquement acceptable est anhydre.

**18.** Composition selon la revendication 17, **caractérisée en ce que** le milieu cosmétiquement acceptable est choisi

parmi les huiles organiques, les silicones, les huiles minérales, les huiles végétales, les cires, les alcanes en $C_5$-$C_{10}$, l'acétone, la méthyléthylcétone, les esters d'acides en $C_1$-$C_{20}$ et d'alcools en $C_1$-$C_8$, le diméthoxyéthane, le diéthoxyéthane, les alcools gras en $C_{10}$-$C_{30}$, les acides gras en $C_{10}$-$C_{30}$, les amides gras en $C_{10}$-$C_{30}$. les esters d'alcool gras en $C_{10}$-$C_{30}$ et leurs mélanges.

**19.** Composition selon l'une quelconque des revendications précédentes, **caractérisée en ce que** la composition contient des inhibiteurs de polymérisation.

**20.** Composition selon la revendication 19, **caractérisée en ce que** les inhibiteurs sont des inhibiteurs de polymérisation anioniques et/ou radicalaires.

**21.** Composition selon la revendication 19 ou 20, **caractérisée en ce que** les inhibiteurs de polymérisation sont choisis parmi le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfonène et leurs mélanges.

**22.** Composition selon l'une quelconque des revendications 19 à 21, **caractérisée en ce que** les inhibiteurs de polymérisation sont présents en des quantités allant de 10 ppm à 20 % par rapport au poids total de la composition.

**23.** Composition selon l'une quelconque des revendications précédentes, **caractérisée en ce que** la composition contient en outre au moins un agent choisi parmi les agents réducteurs, les corps gras, les plastifiants, les adoucissants, les agents anti-mousse, les agents hydratants, les pigments, les argiles, les charges minérales, les filtres UV, les colloïdes minéraux, les peptisants, les solubilisants, les parfums, les conservateurs, les tensioactifs anioniques, non ioniques ou amphotères, les polymères fixants ou non, les polyols, les protéines, des vitamines, les colorants directs ou d'oxydation, et les agents nacrants.

**24.** Composition selon la revendication 23, **caractérisée en ce que** l'agent est encapsulé.

**25.** Utilisation de la composition selon l'une quelconque des revendications précédentes, pour le traitement cosmétique des fibres kératiniques.

**26.** Utilisation selon la revendication 25, en présence d'un agent nucléophile.

**27.** Utilisation selon la revendication 26, **caractérisée en ce que** l'agent nucléophile est choisi parmi les composés moléculaires, les oligomères, les dendrimères ou polymères possédant des fonctions nucléophiles choisies parmi : $R^{iv}{}_2N^-$, $NH_2^-$, $Ph_3C^-$, $R^{iv}{}_3C^-$, $PhNH^-$, pyridine, $ArS^-$, $R^{iv}$-$C{\equiv}C^-$, $R^{iv}S^-$, SH, $R^{iv}O^-$, $R^{iv}{}_2NH$, $ArO^-$, $N_3^-$, $OH^-$, $ArNH_2$, $NH_3$, $I^-$, $Br^-$, $Cl^-$, $R^{iv}COO^-$, $SCN^-$, $R^{iv}OH$, $R^{iv}SH$, $NCO^-$, $CN^-$, $N0_3^-$, $ClO_4^-$ et $H_2O$, Ph représentant le groupe phényle ; Ar représentant un groupe aryle et $R^{iv}$ représentant un groupe alkyle en $C_1$-$C_{10}$.

**28.** Utilisation selon la revendication 27, **caractérisée en ce que** l'agent nucléophile est de l'eau.

**29.** Utilisation selon l'une quelconque des revendications 25 à 28, pour apporter de la brillance aux cheveux.

**30.** Procédé de traitement cosmétique des fibres kératiniques, comprenant l'application d'une composition selon l'une quelconque des revendications précédentes 1 à 24, sur les fibres kératiniques en présence d'un agent nucléophile.

**31.** Procédé selon la revendication 30, **caractérisé en ce que** l'agent nucléophile est choisi parmi les composés moléculaires, les oligomères, les dendrimères ou polymères possédant des fonctions nucléophiles choisies parmi: $R^{iv}{}_2N^-$, $NH_2^-$, $Ph_3C^-$, $R^{iv}{}_3C^-$, $PhNH^-$, pyridine, $ArS^-$, $R^{iv}$-$C{\equiv}C^-$, $R^{iv}S^-$, SH, $R^{iv}O^-$, $R^{iv}{}_2NH$, $ArO^-$, $N_3^-$, $OH^-$, $ArNH_2$, $NH_3$, $I^-$, $Br^-$, $Cl^-$, $R^{iv}COO^-$, $SCN^-$, $R^{iv}OH$, $R^{iv}SH$, $NCO^-$, $CN^-$, $NO_3^-$, $ClO_4^-$ et $H_2O$, Ph représentant le groupe phényle, Ar représentant un groupe aryle et $R^{iv}$ représentant un groupe aryle en $C_1$-$C_{10}$.

**32.** Procédé selon la revendication 31, **caractérisé en ce que** l'agent nucléophile est l'eau.

**33.** Procédé selon l'une quelconque des revendications 30 à 32, **caractérisé en ce que** l'on applique la composition

sur les fibres kératiniques préalablement humidifiées à l'aide d'une solution aqueuse dont le pH a été ajusté à l'aide d'une base, d'un acide ou d'un mélange acide/base.

**34.** Procédé selon l'une quelconque des revendications 30 à 32, **caractérisé en ce que** les fibres kératiniques sont pré-imprégnées à l'aide d'un agent nucléophile différent de l'eau.

**35.** Procédé selon l'une quelconque des revendications 30 à 32, **caractérisé en ce que** les fibres kératiniques sont préalablement réduites avant application de la composition.

**36.** Procédé selon l'une des revendications 30 à 35, **caractérisé en ce que** l'application de la composition est suivie d'un rinçage.

**37.** Procédé selon l'une des revendications 30 à 36, **caractérisé en ce que** les fibres kératiniques sont les cheveux.

**38.** Procédé de traitement cosmétique des fibres kératiniques, comprenant au moins deux étapes, une étape comprenant l'application d'au moins un polymère conducteur tel que défini dans l'une quelconque des revendications 2 à 7, et avec ou sans une éventuelle étape de rinçage intermédiaire, une autre étape d'application d'au moins un monomère électrophile tel que défini dans l'une quelconque des revendications 10 à 15.

**39.** Procédé selon la revendication 38, **caractérisé en ce que** l'application du polymère conducteur se fait avant l'application du monomère électrophile.

**40.** Procédé selon la revendication 38, **caractérisé en ce que** l'application du monomère électrophile se fait avant l'application du polymère conducteur.

**41.** Kit comprenant une première composition contenant au moins un monomère électrophile, et une deuxième composition comprenant dans un milieu cosmétiquement acceptable au moins un polymère conducteur tel que défini dans l'une quelconque des revendications 2 à 7.

Office européen des brevets

# RAPPORT DE RECHERCHE EUROPEENNE

Numéro de la demande
EP 06 29 0508

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 00/45777 A (VAN DIJK, EDDY) 10 août 2000 (2000-08-10) * page 6, ligne 6 - ligne 15; revendications; exemples * ----- | 1-41 | INV. A61K8/44 A61K8/84 A61Q5/06 |
| Y | US 5 362 486 A (NANDAGIRI ET AL) 8 novembre 1994 (1994-11-08) * colonne 4, ligne 11 - ligne 46; revendications; exemples * ----- | 1-41 | |
| Y | WO 03/053380 A (L'OREAL; GIROUD, FRANCK) 3 juillet 2003 (2003-07-03) * page 1, ligne 4 - page 2, ligne 6; revendications; exemples * ----- | 1-41 | |
| X | FR 1 546 631 A (CLAIROL INCORPORATED) 22 novembre 1968 (1968-11-22) * revendications; exemples * ----- | 1-41 | |
| Y | EP 1 498 102 A (L'OREAL) 19 janvier 2005 (2005-01-19) * le document en entier * ----- | 1-41 | DOMAINES TECHNIQUES RECHERCHES (IPC) |
| Y | WO 2004/066968 A (L'OREAL; MOUGIN, NATHALIE; JEGOU, GWENAELLE; SAMAIN, HENRI) 12 août 2004 (2004-08-12) * le document en entier * ----- | 1-41 | A61K |
| Y | WO 2004/043330 A (L'OREAL) 27 mai 2004 (2004-05-27) * page 1, ligne 5 - page 2, ligne 22; revendications; exemples * ----- | 1-41 | |

Le présent rapport a été établi pour toutes les revendications

| Lieu de la recherche | Date d'achèvement de la recherche | Examinateur |
|---|---|---|
| Munich | 7 juillet 2006 | Krattinger, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..........................................................
& : membre de la même famille, document correspondant

4
EPO FORM 1503 03.82 (P04C02)

**ANNEXE AU RAPPORT DE RECHERCHE EUROPEENNE RELATIF A LA DEMANDE DE BREVET EUROPEEN NO.** EP 06 29 0508

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

07-07-2006

| Document brevet cité au rapport de recherche | | Date de publication | Membre(s) de la famille de brevet(s) | | Date de publication |
|---|---|---|---|---|---|
| WO 0045777 | A | 10-08-2000 | AU | 2271400 A | 25-08-2000 |
| US 5362486 | A | 08-11-1994 | AUCUN | | |
| WO 03053380 | A | 03-07-2003 | AU | 2002366749 A1 | 09-07-2003 |
| | | | BR | 0215128 A | 03-11-2004 |
| | | | EP | 1455738 A2 | 15-09-2004 |
| | | | FR | 2833489 A1 | 20-06-2003 |
| | | | JP | 2005513087 T | 12-05-2005 |
| | | | MX | PA04005821 A | 10-09-2004 |
| | | | US | 2003175229 A1 | 18-09-2003 |
| FR 1546631 | A | 22-11-1968 | AUCUN | | |
| EP 1498102 | A | 19-01-2005 | FR | 2857580 A1 | 21-01-2005 |
| | | | JP | 2005048177 A | 24-02-2005 |
| WO 2004066968 | A | 12-08-2004 | AUCUN | | |
| WO 2004043330 | A | 27-05-2004 | AU | 2003292331 A1 | 03-06-2004 |
| | | | EP | 1562538 A2 | 17-08-2005 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9947570 A
- US 6224622 B


### Littérature non-brevet citée dans la description


- Handbook of organic conductive molécules and polymers. Wiley, 1997, vol. 1, 2, 3
- *Can. J. Chem.,* 1986, vol. 64
- *Chem. Mater.,* 1998, vol. 10 (7), 1990-1999
- **RASMUSSEN S.C. ; PICKENS J.C. ; HUTCHISON J.E.** A new général approach to tuning the properties of functionalized polythiophenes : the oxidative polymerization of monosubstituted bithiophenes. *Macromolecules,* 1998, vol. 31, 933-936
- **JERRY MARCH.** Advanced Organic Chemistry. 151-161
- **PR WELLS.** *Prog. Phys. Org. Chem.,* 1968, vol. 6, 111
- **JERRY MARCH.** Advanced Organic Chemistry. 141